Europäisches Patentamt

European Patent Office

Office européen des brevets

(11) Publication number: **0 141 671**
A2

# (12) EUROPEAN PATENT APPLICATION

(21) Application number: **84307649.8**

(22) Date of filing: **06.11.84**

(51) Int. Cl.⁴: **A 61 K 39/10,** A 61 K 37/02

(30) Priority: **07.11.83 US 549384**

(43) Date of publication of application: **15.05.85**
**Bulletin 85/20**

(84) Designated Contracting States: **AT BE CH DE FR GB IT
LI LU NL SE**

(71) Applicant: **SYNTEX (U.S.A.) INC., 3401 Hillview Avenue,
Palo Alto, California 94304 (US)**

(72) Inventor: **Lee, Simon Woon Cheung, 1822 Home Gate
Drive, San Jose California 95148 (US)**

(74) Representative: **Armitage, Ian Michael et al, MEWBURN
ELLIS & CO. 2/3 Cursitor Street, London EC4A 1BQ (GB)**

(54) Bordetella bronchiseptica pilus subunit protein vaccine, composition of matter, and processes for their preparation.

(57) A vaccine derived from B. bronchiseptica pili which has protective activity against atrophic rhinitis and B. pertussis infections is disclosed herein.

EP 0 141 671 A2

ACTORUM AG

-1-

# BORDETELLA BRONCHISEPTICA PILUS SUBUNIT PROTEIN VACCINE, COMPOSITION OF MATTER, AND PROCESSES FOR THEIR PREPARATION

## BACKGROUND OF THE INVENTION

This invention relates to a vaccine for the prevention of atrophic rhinitis caused by B. bronchiseptica and which is effective in preventing B. pertussis infections.

Atrophic rhinitis is a widespread and severe respiratory disease in swine. The disease is characterized by acute rhinitis, followed by chronic atrophy of the turbinate bones. B. bronchiseptica is recognized as the primary pathogen most responsible for the disease syndrome. Newborn piglets are very susceptible to infection. Nasal colonization of baby piglets with B. bronchiseptica usually results in chronic infection and turbinate atrophy leading to snout distortion and reduced rate of weight gain as pigs grow older.

Prevention of B. bronchiseptica induced turbinate atrophy and accelerated nasal clearance of the bacteria by vaccination of swine with a B. bronchiseptica bacterin have been reported. The nature of the immunizing antigen has not been identified.

Vaccines for B. bronchiseptica are disclosed in several literature references, for example, see U.S. Patent 4,203,970 and Goodnow et al, _Journal of Clinical_

_Microbiology_, 6:337-339 (1977). None of these references are drawn to a vaccine derived from pilus protein, particularly pilus subunit proteins.

Pertussis, whooping cough, is an acute infectious disease caused by _Bordetella pertussis_, and characterized by recurrent bouts of spasmodic coughing continued until the breath is exhausted, then ending in a noisy inspiratory stridor (the "whoop") caused by the laryngeal spasm. The lesion is an inflammation of the larynx, trachea, and bronchi.

Though vaccines exist for pertussis, derived from _B. pertussis_, there has not been reported an effective vaccine against pertussis based on antigenic material derived from _B. bronchiseptica_. It has now been discovered that proteins derived from _B. bronchiseptica_ pilus and having the characteristics described herein are capable of imparting protection against _B. pertussis_ infections in addition to their protective activity against _B. bronchiseptica_.

The material responsible for imparting such protection are pilus subunit proteins. The physical structure of proteins are characterized by their primary, secondary, tertiary and quaternary structure. Primary characteristics refer to the amino acid sequence. Secondary and tertiary characteristics refer to the spatial relationship of the peptide chain(s). Proteins comprised of multiple polypeptide chains have an additional organization denoted as the quaternary structure. The word subunit is used to denote the individual chains which make up such polypeptides. Each subunit peptide is individually expressed by the gene, and then assembled into the polypeptide. Subunit peptides have individual structural use or play an individual functional activity in the context of the larger protein. For more detail, see, for example, Biochemistry, The Molecular Basis of Cell Structure and Function, Lehninger, Worth Publishers Inc., New York, New York, 1970. In this invention, the subunit proteins referred to have individual structural function in that each is needed to make up the tertiary structure which comprises the pilus structure.

## SUMMARY OF THE INVENTION

This invention relates to a vaccine for immunization of a mammal against B. bronchiseptica or B. pertussis comprising a protective amount of protein corresponding to one or more pilus subunit proteins derived from B. bronchiseptica. The preferred vaccine will comprise one, two or three subunit proteins having a molecular weight of about 21,000 dalton, 22,000 dalton and 24,000 dalton as determined by sodium dodecyl sulfate-polyacrylamide gel electrophoresis (SDS-PAGE) and by being serologically cross-reactive with all purified pilus preparations from all B. bronchiseptica strains.

In another aspect, this invention is for a composition comprising a substantially pure pilus subunit protein derived from B. bronchiseptica and characterized by having a molecular weight of about 21,000 daltons and/or 22,000 daltons and/or 24,000 daltons as determined by sodium dodecyl sulfate-polyacrylamide gel electrophoresis and being cross-reactive with all purified pilus preparations from all B. bronchiseptica, optionally with a physiologically acceptable excipient.

In another aspect, this invention comprises a method of preparing a pilus subunit protein vaccine for immunization against B. bronchiseptica and B. pertussis which method comprises:

growing a strain of B. bronchiseptica in a suitable medium;

5488K                                                   24170-FF

separating the pili subunit proteins from the medium; and

mixing said proteins with a suitable carrier.

These and other aspects of this invention are set out in greater detail below.

## DETAILED DESCRIPTION OF THE INVENTION

The antigenic factors derived from B. bronchiseptica pilus are subunit proteins. For the purposes of this invention, these antigenic factors are identified by their preparative process, the individual molecular weight of each as determined by sodium dodecyl sulfate-polyacrylamide gel electrophoresis (SDS-PAGE) and their serological activity against B. bronchiseptica pili preparations. These proteins has been shown to be effective in the immunization of pigs against B. bronchiseptica.

In addition, the pilus subunit proteins provide a means for immunizing a mammal against B. pertussis infections.

Microscopic and biochemical analyses indicated that pili from B. bronchiseptica are morphologically and structurally similar. B. bronchiseptica pili are filamentous assemblies of protein subunits. Electron microscopy showed the presence of pili in polyethylene glycol precipitates. The apparent size of a pilus rod was about 3 to 4 micron in diameter.

The pilus-derived proteins responsible for imparting protection are obtained as a mixture upon treating with a precipitant, pili separated from cells and cellular debris. This material, the first precipitant, can be used directly without further purification to prepare a vaccine which can be characterized as a pilus subunit protein vaccine. Suitable precipitants are polyethylene glycols such as polyethylene glycol 6000.

When pili were purified from broth cultures of B. bronchiseptica, as opposed to agar plates, a drastic shift of protein profiles was found. Very little pili were detected by the ELISA test in culture samples obtained during the lag and early exponential phases of growth. Rapid accumulation of pili occurred towards late exponential and early stationary phases. These results may indicate that pili are synthesized during growth but are not assembled and transported to the cell surfaces until the stationary phase.

Depending on the growth conditions and colonial morphology of the organisms, additional purification provides three subunit proteins of three distinct molecular sizes of about 21,000 daltons, 22,000 daltons and 24,000 daltons as determined by sodium dodecyl sulfate-polyacrylamide gel electrophoresis (SDS-PAGE). Two sets of standards were used to characterize these proteins, one set being the proteins K-88, K-99, and 987p E. coli pilus antigens and the other set being the proteins chymotrypsinogen, lactoglobulin, trypsin inhibitor, myoglobulin, ovalbumin and bovine serum albumin

Darkfield microscopy of purified pili showed mostly homogeneous suspensions of needle-like pilus aggregates and the absence of particulate or amorphous structures. Electron microscopy also indicated large amounts of pili.

Using hyperimmune rabbit antipilus sera in an ELISA test, extensive serological cross-reactivities were observed in all purified pilus preparations from all B. bronchiseptica strains. Monoclonal antibody prepared against one preparation of B. bronchiseptica pili also reacts extensively with all other B. bronchiseptica pilus preparations.

Antiserum prepared against each molecular subunit species, whether it is the 21,000 dalton, 22,000 dalton or 24,000 dalton, reacts very strongly with each other.

This is further confirmed by the "Immunoblotting" techniques.

Any B. bronchiseptica strain can be used as a source of pili for atrophic rhinitis vaccines. It is preferrable to use the strain designated NADL 2-9 which was isolated by Dr. D. Croghan, USDA, National Animal Disease Labratory, Ames, Iowa. Other strains are B133 (NVSL, USDA, Ames, Iowa), NALD 7-8 and 110H (Dr. Dave Bemis, University of Tennessee, Knoxville, Tennessee), - 5389, 5998 and 6126 (Dr. Howard Hill, Iowa State University, Ames, Iowa). Strain 5389 is the preferred strain for production of subunit polypeptides for vaccination against B. pertussis infections, ie. whooping cough.

Compositions and vaccines may be prepared with any physiologically acceptable excipient or excipients. Preferably vaccines will be formulated with one or more excipients which have adjuvant activity. For example, the subject proteins may be formulated with Freunds complete and incomplete adjuvant. Alternatively, a mycobacterial cell wall subunit material which has adjuvant activity such as glycodipeptides, e.g. N-acetylmuramyl-L-Ala-D-Igln and its numerous derivatives and analogues, may be incorporated into the vaccine formulation. Other adjuvant excipients such as aluminum hydroxide are also useful. Various and sundry other acceptable formulations which may be used herein are known in the art, such as those set out in Remington's Pharmaceutical Sciences, 16 Ed, A. Osol Ed., Mack Publishing Co., Easton, PA (1980) and other texts and references on pharmaceutical and veterinary formulations.

This invention is further illustrated by the non-limiting preperations and examples which follow.

## EXAMPLE I

. This Example illustrates one method for growing, separating and recovering the pilus subunit proteins referred to above.

Bordetella bronchiseptica was obtained from Dr. David Bemis, University of Tennessee, Knoxville, Tennessee. The culture was originally isolated by Dr. D. Croghan, USDA, National Animal Disease Laboratory, Ames, Iowa and was designated NADL 2-9.

The purity of the strain was determined at the time master seed was prepared. The organisms were evaluated for purity using Tryptic Soy Broth and Thioglycollate Broth. The inoculated flasks were incubated for 14 days and then streaked onto blood agar plates. Representative colonies were examined for morphology and gram stained. They had to result as typical and pure culture of Bordetella bronchiseptica. Biochemical analysis was conducted on isolated colonies.

Master seed cultures and working seed cultures were grown on commercially available Brucella Agar medium. Subcultures and production organisms were grown in production medium. The composition of the production medium was as follows

### Composition of Medium

Base Medium

| | |
|---|---|
| Tryptose | 20.0 g |
| Dextrose | 2.0 g |
| Sodium chloride | 5.0 g |
| Disodium phosphate | 2.5 g |
| Monosodium glutamate | 10.7 g |
| Deionized water q.s. to | 1000.0 ml |

Supplement A

| | |
|---|---|
| L-Cystine | 4.0 g |
| Hydrochloric acid, 4N | 40.0 ml |
| Deionized water q.s. to | 1000.0 ml |

Supplement B

| | |
|---|---|
| Ferrous sulfate (FeSO$_4$.7H$_2$O) | 1.3 g |
| Calcium chloride (CaCl$_2$) | 2.3 g |
| Magnesium chloride (MgCl$_2$.6H$_2$O) | 10.0 g |
| Deionized water q.s. to | 1000.0 ml |

Supplement C

| | |
|---|---|
| Ascorbic acid | 2.0 g |
| Nicotinamide | 1.0 g |
| Sodium acetate | 20.0 g |
| Deionized water q.s. to | 1000.0 ml |

The base medium was steam-sterilized for 15 to 45 minutes. Supplements A, B and C were filter-sterilized. 10 ml of Supplement A, 10 ml of Supplement B, and 10 ml of Supplement C were added to a liter of the base medium previously sterilized and cooled to 40-50°C. Production medium was used within 14 days.

Composition of Brucella Agar Medium

| | |
|---|---|
| Bacto-Peptamin | 20.0 g |
| Bacto-Dextrose | 1.0 g |
| Bacto-Yeast extract | 2.0 g |
| Sodium chloride | 5.0 g |
| Sodium bisulfite | 0.1 g |
| Agar | 15.0 g |
| Deionized water q.s. to | 1000.0 ml |

Brucella Agar Medium was steam-sterilized, dispensed into petri dishes, and used within 30 days.

Working seed cultures and subcultures were grown in 200-2000 ml flasks. Seed cultures used to inoculate the fermenter vessels were grown in 20 liter containers or seed fermenters. Production cultures were grown in 800 liter fermenter vessels.

Cultures are incubated for 10 to 48 hours under controlled conditions. Temperature is maintained at approximately 37°C. Cultures were areated at a constant rate with filtered sterile air and/or oxygen. Dissolved oxygen was maintained at or above 80% saturation. The pH was maintained at 7.1 - 7.6 by additions of propionic acid 2N or Sodium hydroxide 5N. Sterile antifoam

5488K                                                      24170-FF

solution was added as needed to control foaming, not to exceed 6 ml antifoam per liter of medium.

When the culture reaches the desired cell growth, harvest was initiated. The culture is evaluated for typical morphology and growth density. The optical density was determined using a photometer. At the time of the harvest, the optical density of the culture at 560 nm should be 1.5 or greater.

To harvest the culture, it was heated to approximately 60°C for one hour under vigorous agitation and then cooled to room temperature. During this process, pili were detached from the bacteria. Formalin (formaldehyde 37% solution) was added to the culture to make up a final concentration of 0.2% and the culture held at room temperature for 2 or more days. Following inactivation, the culture was transferred to a sterile holding vessel. Merthiolate® (thimerosal) was added to the final product at a concentration of 0.01%.

The inactivated culture may be precipitated by any acceptable method but it is preferred to use polyethylene glycol 6000 (PEG). PEG solution, was added to a concentration of 3%. Sodium chloride was then added to a final concentration of 0.5M. The culture was allowed to settle for at least 24 hours, preferably at 4°-7°C. The precipitated culture was concentrated by continuous flow centrifugation suing Sharples-type equipment. The sediment was then resuspended in Tris buffered saline (0.05M, pH 7.2) and diluted with an equal volume of sterile deionized water containing 0.2% formalin. The resuspended sediment containing antigen and cells was centrifuged again to remove the cells. Following continuous flow centrifugation, the supernatant containing pili was collected. Merthiolate® was then added to 0.01% concentration.

The pili-containing supernatant was then diluted with sterile diluent, Aluminum hydroxide and Merthiolate® in the proportion needed to obtain the desired concentration of the antigen. Diluted material was assayed for antigen content by the ELISA (Enzyme Linked Immunosorbent Assay) test. The pili contents by weight were correlated with the ELISA unitage. One example of such a formulation is:

|  | UNITS/ML | VOLUME (ML) |
|---|---|---|
| Supernatant | 36 U/ml | 50.0 |
| Diluent |  | 112.0 |
| Aluminum hydroxide |  | 18.0 |
| Merthiolate |  | .18 |
| TOTAL | 10 U/ml | 180.18 ml |

The pH was then adjusted to approximately 7.2-7.6 by the addition of sodium hydroxide 5N or hydrochloric acid 4.8N, as required.

## EXAMPLE II

A similiar, but alternative, method for preparing subunit proteins, and the additional step of isolating the three preferred subunit proteins, is exemplified as follows:

An aliquot of B. bronchiseptica, strain NADL 2-9, (0.25 ml of a seed lot) was inoculated on each of 6 Brucella agar plates. These plates were incubated 24 hours at 37°C. Brucella broth, 5 ml, was added to each of the 6 agar plates. The cells were harvested from the plates and resuspended in the 5 ml of Brucella broth. 0.4 ml of the resuspended cells was inoculated for confluent growth on each of a total of 67 Brucella plates. The plates were incubated for 25 hours at 37°C. 10 ml of Tris-buffered saline, pH 7.0, was added to each of the Brucella agar plates. The cells were harvested from the plates in the buffered solution.  Cell suspensions from all plates were combined.

The combined suspension was blended at 12,000 RPM for 15 minutes with an Omnimixer.  Blended cells were centrifuged at 9000 RPM for 30 minutes.  The supernatant was saved and the pellet discarded.  12.4 ml of 30% PEG and 11.8 ml of 4M NaCl per hundred ml of supernatant were added.  The combined solutions were mixed well, then refrigerated  at 4°C for 21 hours.

The antigen precipitate was pelleted by centrifugation at 11,000 RPM for 60 minutes and resuspended in 200 ml of Tris-buffered saline, pH 7.0. The antigen was solubilized by vortexing briefly and mixing for 30 minutes.  The solution was clarified at 9000 RPM for 30 minutes.  This procedure was repeated to give a protein concentrate which was then filtered through a 1.2 μm membrane filter.  The three subunit proteins were separated by sodium dodecyl sulfate-polyacrylamide gel electrophoresis (SDS-PAGE). The subunit proteins' molecular weight was determined  by co-electrophoresis with the K-88, K-99 and 987p in one instance and in another by co-electrophoresis with chymptrypsinogen, lactoglobulin, trypsin inhibitor, myoglobulin, ovalbumin and bovine serum albumin, and were about 21,000, 22,000 and 24,000 daltons respectively. Each protein was recovered from the acrylamide gel and serologic testing was done.  The results of ELISA tests are given in Table 1.

ELISA Titers of Specific Antisera Against
Various Pilus Preparations of B. bronchiseptica

| Antiserum | ELISA Titer | | |
|---|---|---|---|
| Antigen | Anti-21K | Anti-22K | Anti-24K |
| Strain B 133 | 2671 | 337 | 2242 |
| Strain 2-9 | 2623 | 445 | 2746 |
| Strain 7-8 | 2210 | 298 | 1768 |
| Strain 5389 | 2778 | 487 | 2070 |
| Strain 6126 | 2339 | 298 | 2270 |
| Strain 5598 | 2427 | 24 | 704 ⁻ |
| Flagella from Strain 100 NH | 19 | o | 15 |

EXAMPLE III

An study was carried out to evaluate the efficacy of the B. bronchiseptica bacterin prepared in Example I.

The evaluation of immunogenicity was performed in host animals by vaccination of sows and/or offspring and challenge of the pigs. The immunogenicity was determined by comparing nasal turbinate atrophy of vaccinated and non-vaccinated pigs from vaccinated sows to the turbinate atrophy of non-vaccinated pigs from non-vaccinated control sows.

A bacterin prepared as in Example I was diluted 1:2 with a diluent containing 10% Aluminum hydroxide. Immunized sows received two, 2 ml intramuscular vaccinations two weeks apart. The last vaccination was given approximately two weeks prior to farrowing. Immunized piglets received two, 2 ml intramuscular, vaccinations two weeks apart, at 7 and 21 days of age. Following vaccinations, sows and pigs were observed daily for 10 days for local and clinical post-vaccination reactions.

All test pigs were weaned at three weeks of age, transferred to isolation facilities, and then challenged. Pigs were kept in isolation until termination of the test. All piglets were challenged intranasally three days following second vaccination, at 24 days of age. Challenge consisted of 0.5 ml dose in each nostril with an 18 hour culture of <u>Bordetella</u> <u>bronchiseptica</u>, NVSL strain B-133, containing an average of $3.9 \times 10^9$ CFU/ml.

Sows were bled at the time of primary immunization, at the time of second vaccination, and at farrowing. Colostrum samples were also collected during the first 12 hours post-farrowing. Piglets were bled at challenge and at necropsy. Sera were collected and antibody titers determined by the serum agglutination test.

All pigs were sacrificed 60 days following challenge, at about 12 weeks of age. At necropsy, the snout of each pig was sectioned at the level of the first premolar tooth. The degree of turbinate atrophy was determined and scored. The scoring was based on "Scoring system for turbinate atrophy" used at the NVSL, Ames, Iowa. The individual score of each pig and a particular group was statistically analyzed by the Mann-Whitney test and the level of significance between the groups determined.

Scoring system for determination of turbinate atrophy:

0= Normal turbinates

1= Slight distortion of single turbinate

2= Noticeable turbinate atrophy, involving only the ventral turbinates

3= Progressive and severe destruction, involving both turbinates.

4= Complete degeneration of turbinates accompanied by septal malformation.

Test results are summarized as follows:

<u>Immunogenicity Evaluation</u>
<u>Host Animal Test</u>
    <u>Clinical Observation</u>
    Following vaccinations, local or systemic reactions attributable to vaccinations were not observed in any of the vaccinated sows and piglets.

    <u>Turbinate Atrophy Evaluation</u>
    Group I - Vaccinated pigs from vaccinated sows. 87% of pigs (46/53) remained normal or showed only a slight to noticeable single turbinate atrophy (scoring 0, 1, and 2). Severe atrophy was observed in 13% (7/53) pigs (scoring 3). Complete atrophy was not observed. Mean score per pig was 1.52, and 80.5 per group. Results are presented in Table 1 and 2.

    Group II - Non vaccinated pigs from vaccinated sows. Ninety-one percent of the pigs (41/45) remained normal or showed only a slight to noticeable single turbinate atrophy (scoring 0, 1, and 2). Severe atrophy was observed in 9% (4/45) pigs (scoring 3). Complete atrophy was not observed. Mean score per pig was 1.54, and 69.5 per group. Results are presented in Table 1 and 3.

    Group III - Non-vaccinated pigs from non-vaccinated sows. Fifty-four percent of the pigs (31/58) remained normal or showed slight to noticeable turbinate atrophy (scoring 0, 1, and 2). Severe atrophy was observed in 34% (20/58) pigs. Only this group showed complete atrophy (scoring 4) in 12% (7/58) pigs. Mean score per pig was 2.32, and 134.5 per group. The results are presented in Table 1 and 4.

## Serological Response

### · Sows

Prior to vaccination, sows of Groups I, II, and III had a mean antibody titer of 15, 25, and 23, respectively. At the time of the second vaccination, approximately two weeks prior to farrowing, mean antibody titer from sows of Group I and II showed a significant increase to 1024 and 1626, respectively. Antibody titer from sows in Group III was 11.

At farrowing, mean antibody titer of Group I and Group II remained virtually unchanged -- 939 and 1825, respectively. Mean antibody titer of Group III also remained unchanged -- 12. Colostrum samples showed mean antibody titer of 2497 for Group I, 2896 for Group II, and 43 for Group III. Results are presented in Table 5 and summarized in Table 6.

### Pigs

At challenge, mean antibody titer of pigs from Group I (vaccinated sows, vaccinated pigs) was 284. At necropsy the titer declined to 29. Results are presented in Table 7 and summarized in Table 6.

At challenge mean antibody titer of pigs from Group II (vaccinated sow, non vaccinated pigs) was 406. At necropsy the titer declined to 24. Results presented in Table 8 and summarized in Table 6.

Mean antibody titer of pigs from Group III (non vaccinated sows, non vaccinated pigs) was 2 at challenge and 17 at necropsy. Results are presented in Table 9 and summarized in Table 6.

## EXAMPLE III

### Mouse protection study

A determination of the cross-protective activity of the 22K dalton subunit protein against B. pertussis induced whooping cough was carried out as follows. The

5488K 24170-FF

22K protein was obtained by growing two B. bronchiseptica strains, 5389 and NADL 2-9, for 12-16 hours on agar plates and harvested in phosphate-buffered saline solution, pH 6.8. Cell suspensions were individually pooled and blended in a high-speed stirrer at 20,000 for 5 minutes. Each suspension was then clarified by centrifugation at 12,000 X g for 15 minutes and again at 24,000 X g for 15 minutes. Pili were initially purified according to the method of Brinton, C.C. Jr.: The Structure, Function, Synthesis and genetic control of bacterial pili and a Molecular Module for DNA and RNA transport in Gram-negative Bacteria. Trans NY Acad. Sci., 27:2003-2054, (1965). Magnesium chloride was added to a final concentration of 0.1M, and the solution was allowed to stand overnight at 4°C. Pili were pelleted by centrifugation at 12,000 X g for 60 minutes. The 5389 strain gave primarily 22K dalton pili with a very minor amount (less than 10%) of 24K dalton protein. Strain NADL 2-9 gave about equal amounts of 21K and 24K dalton protein with a very minor amount (about 10%) of 22K dalton subunit protein. The molecular weight distribution of these pili subunit proteins was confirmed by SDS-PAGE.

Infant BALB/C mice were immunized intraperitoneally with 100 µl of vaccine containing 8 µg of Bordetella bronchiseptica pilus from one or the other of the two strains and 20 µg of aluminum hydroxide gel at 6 days of age and again at 12 days of age. Mice in the control groups were also vaccinated with either B. pertussis or tetanus toxoid. Seven days after the second vaccination, mice were challenged by placing the animals in an aerosol chamber for 45 minutes. Bacterial aerosol was generated using a culture suspension of Bordetella pertussis strain 18323 (2.6 x 10 CFU/ml). A "zero time" infectivity dose of B. pertussis was determined to be 1 x 10 CFU/mouse by·

counting number of viable B. pertussis present in the mouse lung. Body weight gain, white blood cell count and survivors were monitored for 23 days post-challenge.

| Test Group | Vaccine received | Survivors/Total | % Survivors |
|---|---|---|---|
| Noninfected control | none | 6/6 | 100 |
| Vaccinated control | B. pertussis pilus, 14 µg | 0/14 | 0 |
| Vaccinated control | B. pertussis pilus, 14 µg | 13/13 | 100 |
| Test | B. bronchi. pilus strain 5389, 8 µg | 6/7 | 86 |
| Test | B. bronchi. pilus strain NADL 2-9, 8 µg | 1/6 | 17 |

------------------------------------------------------------------

Discussion

Mice vaccinated with B. bronchiseptica pilus of 22K daltons (those treated with the 5389 strain derived pili) were well protected against aerosol challenge of virulent B. pertussis at 8 µg. Those vaccinated with 21K and 24K subunit proteins with a minor amount of 22K protein showed some level of protection. Mice immunized with 8µg of protein from the 5389 strain showed levels of protection about equal to mice immunized with B. pertussis pilus. Mice vaccinated with pili from the 5389 strain and B. pertussis showed normal body weight gain and normal level of white blood cell count when compared with noninfected animals as illustrated by Figures I and II.

Mice vaccinated with NADL 2-9 derived pilus protein showed moderate protection against B. pertussis infection. This group showed white blood cell counts and

5488K                                                        24170-FF

body weight gains which were intermediate to those figures for strain 5389 and B. pertussis treated animals and the tetanus toxoid treated animals. This indicates there is some protective protein present in the NADL 2-9 pili isolate though greater amounts of this isolate apparently would be necessary to give full protective activity.

Vaccination of mice with tetanus toxoid, an immunologically unrelated antigen, was not protective. All animals in this group died within 16 days after exposure to B. pertussis.

These results demonstrate the protective immunogenicity of Bordetella bronchiseptica pilus against B. pertussis infection.

## EXAMPLE IV

The 22K dalton protein was purified and its amino acid composition determined as follows. B. bronchiseptica, strain 5389, was grown up in a fermenter and processed to recover the pili as described in Example I. Pilus subunit proteins were further separated by SDS-polyacrylamide gel electrophoresis (PAGE) and localized on the gel slab by staining with 0.25 M KCl (D. Hager and R. Burgess, Anal. Biochem., 109:76-86 (1980). Gel strips containing the 22K dalton protein were excised and passed through a syringe. The protein was eluted by soaking the gel in a small volume of buffer containing 0.1 M ammonium bicarbonate and 0.1% SDS. After 3 to 12 house of incubation at 37°C, acrylamide fragments were sedimented by low speed contribution and the supernatant was collected. For optimal recovery, up to four elutions were performed. Supernatants were pooled and adjusted to 0.4 M ammonium bicarbonate concentration. Nine volumes of anhydrous acetone were added. Flocculent precipitates of inorganic salt-protein complexes were collected by centrifugation and dried by blowing with nitrogen. The precipitate was rehydrated with water. Protein concentration and purity of each fraction was determined by analytical SDS-PAGE. Ammonium bicarbonate was removed by lyophilization and the protein was then dissolved and hydrolyzed in hydrochloric acid and subjected to amino acid analysis.

Alternatively, the pilus subunit protein bands may be visualized by coomassie blue staining to facilitate excision. SDS and coomassie blue stain may be extracted with a solvent containing acetone, trimethylamine, acieic acid and water (Henderson, L. & E.S. Oroszlan, Anal. Biochem., 93:153-157). Protein precipitates were collected and prepared for amino acid analysis as described in the preceeding paragraph.

The amino acid composition was determined to be:

Amino acid composition of Bordetella Bronchiseptica 22,000 dalton pilus subunit protein (number of residues per subunit molecule)

| aspartic acid (1) | 26 |  |
|---|---|---|
| threonine | 22 |  |
| serine | 15 |  |
| glutamic acid (2) | 13 |  |
| proline | 14 |  |
| glycine | 20 |  |
| alanine | 19 |  |
| cystine/cysteine | -- | (3) |
| valine | 16 |  |
| methionine | 1 |  |
| isoleucine | 13 |  |
| leucine | 11 |  |
| tyrosine | 10 |  |
| phenylalanine | 5 |  |
| histidine | 2 |  |

0141671

| | |
|---|---|
| lysine | 13 |
| tryptophan | 2 |
| arginine | 5 |

(1) aspartic acid plus asparagine

(2) glutamic acid plus glutamate

(3) present but not quantified

The method used was:

Amino acid analysis

Proteins were dissolved in 6 N HCl and samples were degassed by repeated freezing and thawing under vacuum. Hydrolysis was carried out at 110°C for 22 hours. HCl was removed and hydrolysate dried by rotary evaporation and analyzed using a Beckman (Trade Mark) amino acid analyzer. Calculations for the amino acid contents were carried out as described by Moore and Stein*. The approximate corrections applied for the decomposition of amino acids were 5 percent for threonine and tyrosine, and 10 percent for serine. Tryptophan was determined spectrophotometrically by the method of Edelhoch**.

* Moore, S. and Stein, W. 1963 'Chromatographic determination of amino acid by the use of automatic recording equipment'. Methods in enzymology. Vol 6, P. 819-831.

** Edelhoch, H. 1967. 'Spectroscopic determination of tryptophan and tyrosine in protein'. Biochem 6:1948-1954.

Table 1
BORDETELLA BRONCHISEPTICA BACTERIN
HOST ANIMAL EFFICACY STUDY
MEAN SCORES AND FREQUENCY DISTRIBUTION OF TURBINATE ATROPHY

| Group | Treatment | No. of Pigs Per Group | Percent and Number ( ) of Pigs Showing Various Turbinate Scores * | | | | | Mean Score | |
|---|---|---|---|---|---|---|---|---|---|
| | | | 0 | 1 | 2 | 3 | 4 | Per Group | Per Pig |
| I | Vaccinated Sows | | | | | | | | |
| | Vaccinated Pigs | 53 | 8 ( 4) | 28 (15) | 51 (27) | 13 ( 7) | 0 ( 0) | 80.5 | 1.52 |
| II | Vaccinated Sows | | | | | | | | |
| | Non Vaccinated Pigs | 45 | 9 ( 4) | 20 ( 9) | 62 (28) | 9 ( 4) | 0 ( 0) | 69.5 | 1.54 |
| III | Non Vaccinated Sows | | | | | | | | |
| | Non Vaccinated Pigs | 58 | 0 ( 0) | 7 ( 4) | 47 (27) | 34 (20) | 12 ( 7) | 134.5 | 2.32 |

Turbinate Atrophy Scoring

0 Normal turbinates
1 Slight turbinates atrophy
2 Very noticeable turbinate atrophy involving only the ventral turbinates
3 Progressive destruction of both turbinates
4 Nearly complete destruction of the turbinates and septal malformation

## TABLE 2
## BORDETELLA BRONCHISEPTICA BACTERIN
## HOST ANIMAL EFFICACY STUDY
## TURBINATE ATROPHY EVALUATION

0141671

### Vaccinated Sows, Vaccinated Pigs (Group I)

| Sow No. and Vaccination Dates | Pig No. | Date (1983) Farrowed | Date (1983) Challenged | Date (1983) Necropsy | Turbinate Atrophy Score Left | Turbinate Atrophy Score Right | Turbinate Atrophy Score Total |
|---|---|---|---|---|---|---|---|
| 985 3-31 4-21 | 571 | 4-21 | 5-16 | 8-8 | 1 | 0 | 1 |
| | 572 | | | | 1 | 0 | 1 |
| | 573 | | | | 0 | 0 | 0 |
| | 574 | | | | 0 | 0 | 0 |
| | 575 | | | | 0 | 1 | 1 |
| | 576 | | | | 0 | 0 | 0 |
| | 577 | | | | 3 | 2 | 5 |
| | 578 | | | | 1 | 1 | 2 |
| 966 3-31 4-21 | 579 | 5-6 | 5-31 | 8-8 | 2 | 2 | 4 |
| | 580 | | | | 2 | 2 | 4 |
| | 581 | | | | 2 | 2 | 4 |
| | 582 | | | | 2 | 2 | 4 |
| | 583 | | | | 2 | 1 | 3 |
| | 584 | | | | 2 | 2 | 4 |
| 967 3-31 4-21 | 585 | 5-9 | 6-2 | 8-8 | 1 | 0 | 1 |
| | 586 | | | | 3 | 3 | 6 |
| | 587 | | | | 3 | 3 | 6 |
| | 588 | | | | 3 | 3 | 6 |
| | 589 | | | | 1 | 1 | 2 |
| 989 3-31 4-21 | 590 | 5-11 | 6-2 | 8-8 | 2 | 2 | 4 |
| | 591 | | | | 2 | 2 | 4 |
| | 592 | | | | 2 | 2 | 4 |
| 972 3-31 4-21 | 609 | 5-12 | 6-3 | 8-8 | 3 | 3 | 6 |
| | 610 | | | | 3 | 1 | 4 |
| | 611 | | | | 1 | 1 | 2 |
| | 612 | | | | 2 | 1 | 3 |
| | 613 | | | | 3 | 2 | 5 |
| | 614 | | | | 2 | 2 | 4 |
| | 615 | | | | 2 | 2 | 4 |
| | 616 | | | | 2 | 2 | 4 |
| | 617 | | | | 1 | 2 | 3 |
| | 618 | | | | 1 | 2 | 3 |
| 970 3-31 5-9 | 619 | 5-13 | 6-7 | 8-22 | 2 | 2 | 4 |
| | 620 | | | | 2 | 2 | 4 |
| | 621 | | | | 3 | 2 | 5 |
| | 622 | | | | 1 | 1 | 2 |
| | 623 | | | | 1 | 1 | 2 |
| | 624 | | | | 1 | 2 | 3 |
| 969 3-31 4-21 | 594 | 5-14 | 6-7 | 8-22 | 1 | 1 | 2 |
| | 595 | | | | 1 | 1 | 2 |
| | 596 | | | | 1 | 0 | 1 |
| | 597 | | | | 0 | 0 | 0 |
| | 598 | | | | 2 | 2 | 4 |
| | 599 | | | | 1 | 1 | 2 |
| | 600 | | | | 1 | 2 | 3 |
| | 601 | | | | 0 | 1 | 1 |
| | 602 | | | | 2 | 2 | 4 |
| | 603 | | | | 2 | 1 | 3 |
| 990 3-31 4-21 | 604 | 5-16 | 6-7 | 8-22 | 0 | 1 | 1 |
| | 605 | | | | 2 | 1 | 3 |
| | 606 | | | | 2 | 1 | 3 |
| | 607 | | | | 2 | 2 | 4 |
| | 608 | | | | 2 | 2 | 4 |
| Total Sows 8    Pigs 53 | | | | Scores | 84 | 77 | 161 |

Average score per pig = 80.5/53 = 1.52

-23-

TABLE 3
BORDETELLA BRONCHISEPTICA BACTERIN
HOST ANIMAL EFFICACY STUDY
TURBINATE ATROPHY EVALUATION
Vaccinated Sows, Non Vaccinated Pigs (Group II)

| Sow No. and Vaccination Dates | Pig No. | Date (1983) | | | Turbinate Atrophy Score | | |
|---|---|---|---|---|---|---|---|
| | | Farrowed | Challenged | Necropsy | Left | Right | Total |
| 988 | 683 | 5-22 | 6-14 | 8-29 | 3 | 3 | 6 |
| 3-31 | 684 | | | | 1 | 2 | 3 |
| 5-9 | 685 | | | | 2 | 2 | 4 |
| | 686 | | | | 0 | 0 | 0 |
| | 687 | | | | 1 | 1 | 2 |
| | 688 | | | | 2 | 2 | 4 |
| | 689 | | | | 2 | 2 | 4 |
| | 690 | | | | 2 | 2 | 4 |
| | 691 | | | | 1 | 0 | 1 |
| 974 | 692 | 5-22 | 6-14 | 8-29 | 2 | 2 | 4 |
| 3-31 | 693 | | | | 0 | 0 | 0 |
| 5-9 | 694 | | | | 0 | 0 | 0 |
| | 695 | | | | 1 | 1 | 2 |
| | 696 | | | | 2 | 1 | 3 |
| | 697 | | | | 2 | 1 | 3 |
| | 698 | | | | 0 | 1 | 1 |
| 973 | 699 | 5-28 | 6-21 | 8-29 | 1 | 2 | 3 |
| 4-21 | 700 | | | | 1 | 1 | 2 |
| 5-16 | 701 | | | | 2 | 2 | 4 |
| | 702 | | | | 2 | 2 | 4 |
| | 703 | | | | 1 | 1 | 2 |
| | 704 | | | | 2 | 2 | 4 |
| 978 | 705 | 6-1 | 6-23 | 8-29 | 2 | 2 | 4 |
| 3-31 | 706 | | | | 0 | 0 | 0 |
| 4-21 | 707 | | | | 2 | 2 | 4 |
| | 708 | | | | 2 | 3 | 5 |
| | 709 | | | | 1 | 2 | 3 |
| | 710 | | | | 2 | 2 | 4 |
| | 711 | | | | 3 | 2 | 5 |
| | 712 | | | | 1 | 2 | 3 |
| 976 | 713 | 6-1 | 6-23 | 8-29 | 2 | 2 | 4 |
| 3-31 | 714 | | | | 1 | 2 | 3 |
| 5-16 | 715 | | | | 1 | 2 | 3 |
| | 716 | | | | 1 | 1 | 2 |
| | 717 | | | | 0 | 1 | 1 |
| | 718 | | | | 1 | 1 | 2 |
| | 719 | | | | 2 | 2 | 4 |
| 981 | 720 | 6-1 | 6-23 | 8-29 | 2 | 3 | 5 |
| 4-21 | 721 | | | | 2 | 2 | 4 |
| 5-16 | 722 | | | | 2 | 2 | 4 |
| | 723 | | | | 2 | 2 | 4 |
| | 724 | | | | 2 | 2 | 4 |
| | 725 | | | | 2 | 2 | 4 |
| | 726 | | | | 1 | 2 | 3 |
| | 727 | | | | 2 | 2 | 4 |
| Total Sows 6 | | Pigs 45 | | Scores | 66 | 73 | 139 |

Average score per pig = 69.5/45 = 1.54

# TABLE 4

0141671

## BORDETELLA BRONCHISEPTICA BACTERIN
## HOST ANIMAL EFFICACY STUDY
## TURBINATE ATROPHY EVALUATION

Non Vaccinated Sows, Non Vaccinated Pigs (Group III)

| Sow No. | Pig No. | Date (1983) Farrowed | Challenged | Necropsy | Turbinate Atrophy Score Left | Right | Total |
|---|---|---|---|---|---|---|---|
| 987 | 625 | 5-2 | 5-27 | 8-8 | 5 | 3 | 8 |
|  | 626 |  |  |  | 2 | 2 | 4 |
|  | 627 |  |  |  | 2 | 2 | 4 |
|  | 628 |  |  |  | 2 | 2 | 4 |
|  | 629 |  |  |  | 3 | 3 | 6 |
|  | 630 |  |  |  | 2 | 3 | 5 |
|  | 631 |  |  |  | 0 | 1 | 1 |
| 975 | 632 | 5-2 | 5-27 | 8-8 | 3 | 2 | 5 |
|  | 633 |  |  |  | 3 | 2 | 5 |
|  | 634 |  |  |  | 2 | 2 | 4 |
|  | 635 |  |  |  | 2 | 2 | 4 |
|  | 636 |  |  |  | 3 | 3 | 6 |
|  | 637 |  |  |  | 1 | 0 | 1 |
|  | 638 |  |  |  | 3 | 2 | 5 |
|  | 639 |  |  |  | 2 | 2 | 4 |
| 971 | 640 | 5-12 | 6-3 | 8-8 | 2 | 2 | 4 |
|  | 641 |  |  |  | 3 | 3 | 6 |
|  | 642 |  |  |  | 2 | 2 | 4 |
|  | 643 |  |  |  | 2 | 2 | 4 |
|  | 644 |  |  |  | 3 | 2 | 5 |
|  | 645 |  |  |  | 2 | 3 | 5 |
|  | 646 |  |  |  | 3 | 2 | 5 |
|  | 647 |  |  |  | 3 | 3 | 6 |
| 986 | 648 | 5-11 | 6-2 | 8-8 | 1 | 2 | 3 |
|  | 649 |  |  |  | 2 | 2 | 4 |
|  | 650 |  |  |  | 2 | 2 | 4 |
|  | 651 |  |  |  | 3 | 3 | 6 |
| 977 | 663 | 5-9 | 6-2 | 8-8 | 3 | 3 | 6 |
|  | 664 |  |  |  | 1 | 2 | 3 |
|  | 665 |  |  |  | 3 | 4 | 7 |
|  | 666 |  |  |  | 1 | 0 | 1 |
|  | 667 |  |  |  | 3 | 0 | 3 |
| 984 | 652 | 5-14 | 6-7 | 8-22 | 2 | 2 | 4 |
|  | 653 |  |  |  | 1 | 3 | 4 |
|  | 654 |  |  |  | 5 | 2 | 7 |
|  | 655 |  |  |  | 2 | 2 | 4 |
|  | 656 |  |  |  | 2 | 2 | 4 |
|  | 657 |  |  |  | 2 | 2 | 4 |
|  | 658 |  |  |  | 3 | 3 | 6 |
|  | 659 |  |  |  | 3 | 3 | 6 |
|  | 660 |  |  |  | 2 | 2 | 4 |
|  | 661 |  |  |  | 3 | 3 | 6 |
|  | 662 |  |  |  | 2 | 3 | 5 |
| 968 | 668 | 5-14 | 6-7 | 8-22 | 3 | 5 | 8 |
|  | 669 |  |  |  | 4 | 4 | 8 |
|  | 670 |  |  |  | 5 | 2 | 7 |
|  | 671 |  |  |  | 1 | 1 | 2 |
|  | 672 |  |  |  | 2 | 1 | 3 |
|  | 673 |  |  |  | 3 | 5 | 8 |
|  | 674 |  |  |  | 3 | 3 | 6 |
| 980 | 675 | 5-19 | 6-10 | 8-22 | 2 | 2 | 4 |
|  | 676 |  |  |  | 1 | 3 | 4 |
|  | 677 |  |  |  | 1 | 2 | 3 |
|  | 678 |  |  |  | 2 | 3 | 5 |
|  | 679 |  |  |  | 1 | 2 | 3 |
|  | 680 |  |  |  | 2 | 3 | 5 |
|  | 681 |  |  |  | 2 | 2 | 4 |
|  | 682 |  |  |  | 2 | 1 | 3 |

Total Sows, 8          Pigs  58          Scores          135          134          269

Average score per pig = 134.5/58 = 2.32

TABLE 5

BORDETELLA BRONCHISEPTICA BACTERIN
HOST ANIMAL EFFICACY STUDY
SEROLOGICAL RESPONSE IN SOWS

| Group No. | Sow No. | Dates Vac. | Antibody Titers[a] | | | |
|-----------|---------|------------|------|------|------|------|
| | | | Vaccination | | Post 2nd | |
| | | | Pre | Post 1st | At Farrow | Colostrum |
| | 985 | 3-31-83 4-21-83 | 16 | 512 | 1024 | NS[b] |
| | 966 | 3-31-83 4-21-83 | 8 | 2048 | 2048 | 4096 |
| | 967 | 3-31-83 4-21-83 | 16 | 2048 | 1024 | 4096 |
| I | 989 | 3-31-83 4-21-83 | 8 | 1024 | 1024 | 4096 |
| | 972 | 3-31-83 4-21-83 | 32 | 1024 | 512 | 2048 |
| | 970 | 3-31-83 5-9-83 | 8 | 256 | 512 | 512 |
| | 969 | 3-31-83 4-21-83 | 32 | 2048 | 1024 | 4096 |
| | 990 | 3-31-83 4-21-83 | 16 | 1024 | 1024 | 2048 |
| Geometric Mean Titer | | | 15 | 1024 | 939 | 2497 |
| | 988 | 3-31-83 5-9-83 | 16 | 1024 | 4096 | 4096 |
| | 974 | 3-31-83 5-9-83 | 128 | 4096 | 4096 | 4096 |
| II | 973 | 4-21-83 5-16-83 | 32 | 2048 | 4096 | 8192 |
| | 978 | 3-31-83 4-21-83 | 64 | 4096 | 512 | 2048 |
| | 976 | 3-31-83 5-16-83 | 4 | 512 | 1024 | 4096 |
| | 981 | 4-21-83 5-16-83 | 16 | 1024 | 1024 | 512 |
| Geometric Mean Titer | | | 25 | 1626 | 1825 | 2896 |
| | 987 | NA[c] | 16 | 8 | 4 | 32 |
| | 975 | NA | 32 | 32 | 32 | 64 |
| | 971 | NA | 16 | 2 | 4 | 16 |
| III | 986 | NA | 32 | 16 | 16 | 32 |
| | 977 | NA | 16 | 16 | 16 | 64 |
| | 984 | NA | 64 | .16 | 32 | 128 |
| | 968 | NA | 16 | 8 | 8 | 32 |
| | 980 | NA | 16 | 16 | 16 | NS |
| Geometric Mean Titer | | | 23 | 11 | 12 | 43 |

[a] Antibody titer reported as reciproal of dilution
[b] No sample
[c] Not applicable

0141671

## TABLE 6
### BORDETELLA BRONCHISEPTICA BACTERIN
### HOST ANIMAL EFFICACY STUDY.

## SEROLOGICAL RESPONSE -- MEAN ANTIBODY TITER
## SUMMARY

| Group | Treatment | Vaccination | | | | | |
|---|---|---|---|---|---|---|---|
| | | Sows | | | | Pigs | |
| | | Pre | Post 1 | Farrow | Colostrum | At Challenge | At Necropsy |
| I | Vaccinated Sows Vaccinated Pigs | 15 | 1024 | 939 | 2497 | 284 | 29 |
| II | Vaccinated Sows Non Vaccinated Pigs | 25 | 1626 | 1825 | 2896 | 406 | 24 |
| III | Non Vaccinated Sows Non Vaccinated Pigs | 23 | 11 | 12 | 43 | 2 | 17 |

# TABLE 7

## BORDETELLA BRONCHISEPTICA BACTERIN
## HOST ANIMAL EFFICACY STUDY
## SEROLOGICAL RESPONSE IN PIGS
### Vaccinated Sows, Vaccinated Pigs (Group I)

| Animal No. | | Antibody Titers[a] | |
|---|---|---|---|
| Sow | Pig | At Challenge | At Necropsy |
| 985 | 571 | 256 | 32 |
| | 572 | 512 | 16 |
| | 573 | 256 | 32 |
| | 574 | 512 | 16 |
| | 575 | 256 | 16 |
| | 576 | 256 | 16 |
| | 577 | 1024 | 32 |
| | 578 | 256 | 16 |
| 966 | 579 | 512 | 16 |
| | 580 | 256 | 16 |
| | 581 | 512 | 16 |
| | 582 | 256 | 16 |
| | 583 | 256 | 16 |
| | 584 | 256 | 16 |
| 967 | 585 | 128 | 64 |
| | 586 | 64 | 32 |
| | 587 | 256 | 32 |
| | 588 | 256 | 64 |
| | 589 | 256 | 128 |
| 989 | 590 | 512 | 16 |
| | 591 | 256 | 16 |
| | 592 | 256 | 16 |
| 972 | 609 | 256 | 32 |
| | 610 | 512 | 16 |
| | 611 | 256 | 64 |
| | 612 | 256 | 32 |
| | 613 | 256 | 8 |
| | 614 | 512 | 256 |
| | 615 | 256 | 128 |
| | 616 | 256 | 16 |
| | 617 | 512 | 16 |
| | 618 | 512 | 16 |
| 970 | 619 | 64 | 16 |
| | 620 | 512 | 64 |
| | 621 | 512 | 32 |
| | 622 | 128 | 64 |
| | 623 | 256 | 16 |
| | 624 | 64 | 128 |
| 969 | 594 | 512 | 8 |
| | 595 | 512 | 32 |
| | 596 | 512 | 16 |
| | 597 | 256 | 16 |
| | 598 | 128 | 8 |
| | 599 | 512 | 32 |
| | 600 | 128 | 128 |
| | 601 | 512 | 32 |
| | 602 | 512 | 8 |
| | 603 | 512 | 16 |
| 990 | 604 | 256 | 128 |
| | 605 | 512 | 32 |
| | 606 | 256 | 512 |
| | 607 | 64 | 64 |
| | 608 | 128 | 32 |
| Geometric Mean Titer | | 284 | 29 |

a  Antibody titer reported as reciprocal of serum dilution

-28-

## TABLE 8
### BORDETELLA BRONCHISEPTICA BACTERIN
### HOST ANIMAL EFFICACY STUDY
### SEROLOGICAL RESPONSE IN PIGS

Vaccinated Sows, Non Vaccinated Pigs (Group II)

| Animal No. | | Antibody Titers[a] | |
|---|---|---|---|
| Sow | Pig | At Challenge | At Necropsy |
| 988 | 683 | 512 | 16 |
|  | 684 | 512 | 32 |
|  | 685 | 1024 | 32 |
|  | 686 | 1024 | 32 |
|  | 687 | 1024 | 16 |
|  | 688 | 1024 | 16 |
|  | 689 | 1024 | 32 |
|  | 690 | 1024 | 32 |
|  | 691 | 512 | 32 |
| 974 | 692 | 256 | 16 |
|  | 693 | 256 | 16 |
|  | 694 | 256 | 32 |
|  | 695 | 128 | 16 |
|  | 696 | 1024 | 16 |
|  | 697 | 512 | 8 |
|  | 698 | 64 | 8 |
| 973 | 699 | 1024 | 32 |
|  | 700 | 1024 | 32 |
|  | 701 | 1024 | 32 |
|  | 702 | 512 | 32 |
|  | 703 | 1024 | 32 |
|  | 704 | 512 | 32 |
| 978 | 705 | 256 | 16 |
|  | 706 | 256 | 64 |
|  | 707 | 256 | 64 |
|  | 708 | 128 | 8 |
|  | 709 | 128 | 32 |
|  | 710 | 256 | 16 |
|  | 711 | 512 | 16 |
|  | 712 | 256 | 16 |
| 976 | 713 | 512 | 32 |
|  | 714 | 512 | 16 |
|  | 715 | 512 | 32 |
|  | 716 | 512 | 32 |
|  | 717 | 512 | 32 |
|  | 718 | 512 | 32 |
|  | 719 | 256 | 16 |
| 981 | 720 | 256 | 8 |
|  | 721 | 128 | 16 |
|  | 722 | 512 | 32 |
|  | 723 | 256 | 16 |
|  | 724 | 256 | 2048 |
|  | 725 | 256 | 8 |
|  | 726 | 256 | 16 |
|  | 727 | 256 | 16 |
| Geometric Mean Titer | | 406 | 24 |

[a] Antibody titer reported as reciprocal of serum dilution

## TABLE 9
### BORDETELLA BRONCHISEPTICA BACTERIN
### HOST ANIMAL EFFICACY STUDY
### SEROLOGICAL RESPONSE IN PIGS

0141671

Non Vaccinated Sows, Non Vaccinated Pigs (Group III)

| Animal No. | | Antibody Titers[a] | |
|---|---|---|---|
| Sow | Pig | At Challenge | At Necropsy |
| 987 | 625 | 2 | 16 |
| | 626 | 2 | 8 |
| | 627 | 2 | 32 |
| | 628 | 2 | 16 |
| | 629 | 2 | 16 |
| | 630 | 2 | 4 |
| | 631 | 2 | 8 |
| 975 | 632 | 2 | 16 |
| | 633 | 2 | 8 |
| | 634 | 2 | 8 |
| | 635 | 2 | 32 |
| | 636 | 2 | 32 |
| | 637 | 2 | 16 |
| | 638 | 2 | 32 |
| | 639 | 2 | 16 |
| 971 | 640 | 2 | 64 |
| | 641 | 2 | 16 |
| | 642 | 2 | 16 |
| | 643 | 2 | 32 |
| | 644 | 2 | 2 |
| | 645 | 2 | 4 |
| | 646 | 2 | 16 |
| | 647 | 2 | 16 |
| 986 | 648 | 2 | 32 |
| | 649 | 2 | 32 |
| | 650 | 2 | 16 |
| | 651 | 2 | 32 |
| 977 | 663 | 2 | 64 |
| | 664 | 2 | 64 |
| | 665 | 2 | 16 |
| | 666 | 2 | 32 |
| | 667 | 2 | 32 |
| 984 | 652 | 2 | 8 |
| | 653 | 2 | 16 |
| | 654 | 4 | 16 |
| | 655 | 2 | 8 |
| | 656 | 2 | 16 |
| | 657 | 2 | 8 |
| | 658 | 2 | 64 |
| | 659 | 2 | 4 |
| | 660 | 2 | 16 |
| | 661 | 2 | 16 |
| | 662 | 2 | 4 |
| 968 | 668 | 2 | 8 |
| | 669 | 2 | 8 |
| | 670 | 2 | 4 |
| | 671 | 2 | 128 |
| | 672 | 2 | 32 |
| | 673 | 2 | 8 |
| | 674 | 2 | 16 |
| 980 | 675 | 2 | 64 |
| | 676 | 2 | 64 |
| | 677 | 2 | 32 |
| | 678 | 2 | 16 |
| | 679 | 2 | 8 |
| | 680 | 2 | 32 |
| | 681 | 2 | 16 |
| | 682 | 2 | 32 |
| Geometric Mean Titer | | 2 | 17 |

a  Antibody titer reported as reciprocal of serum dilution

0141671

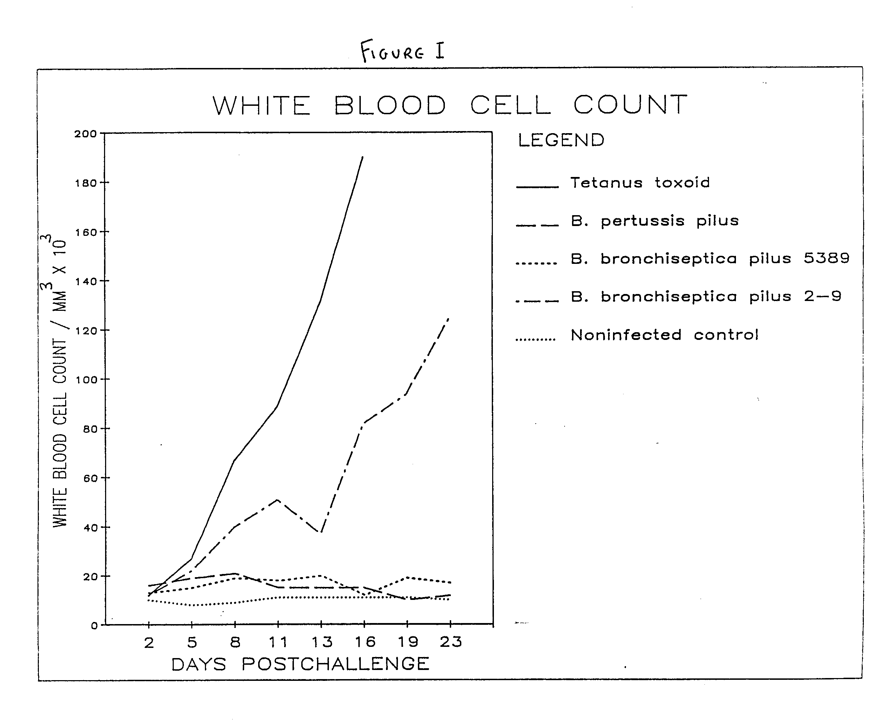

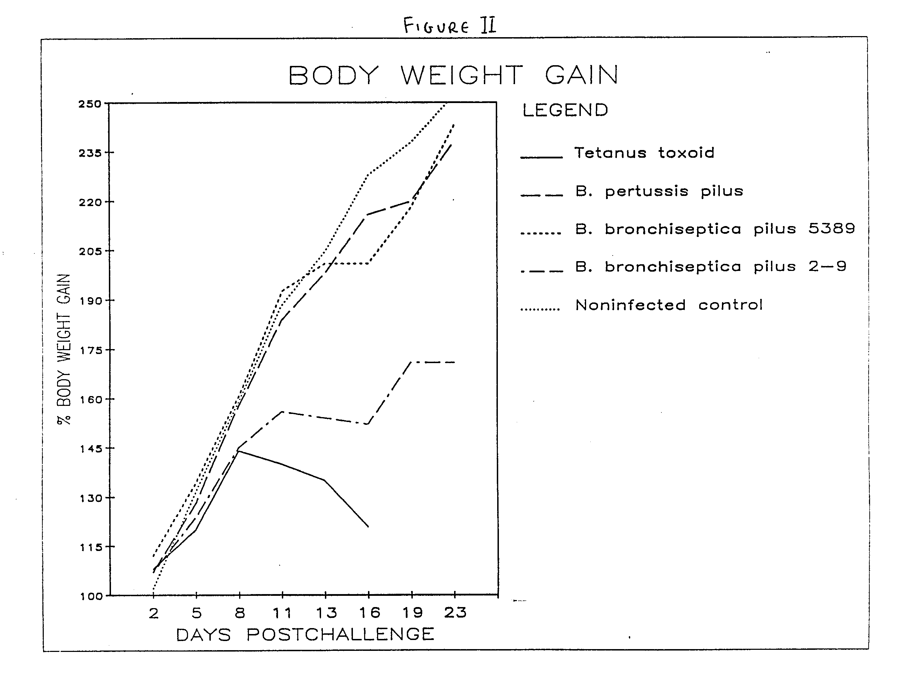

CLAIMS:

1.    A vaccine comprising protein corresponding to one or more pilus subunit proteins derived from B. bronchiseptica.

2.    A vaccine of Claim 1 for immunization against B. bronchiseptica.

3.    A vaccine of Claim 1 or Claim 2 wherein said subunit protein is characterized by having a molecular weight of about 21,000 dalton and/or 22,000 dalton and/or 24,000 dalton as determined by sodium dodecyl sulfate-polyacrylamide gel electrophoresis (SDS-PAGE) and by being serologically cross-reactive with all purified pilus preparations from all B. bronchiseptica strains.

4.    The vaccine of any one of claims 1,2 and 3 wherein said subunit protein is derived from the B. bronchiseptica strain NADL 2-9.

5.    A vaccine of Claim 1 for immunization against B. pertussis induced whooping cough.

6.    The vaccine of Claim 5 wherein the subunit protein has a molecular weight of about 22K as determined by sodium dodecyl sulfate-polyacrylamide gel electrophoresis (SDS-PAGE).

7.    A vaccine of Claim 5 or 6 wherein said subunit protein is derived from the B. bronchiseptica strain 5389.

8. A composition of matter comprising a substantially pure pilus subunit protein derived from B. bronchiseptica and characterized by having a molecular weight of about 21,000 daltons as determined by sodium dodecyl sulfate-polyacrylamide gel electrophoresis and being cross-reactive with all purified pilus preparations from all B. bronchiseptica.

9. A composition of matter comprising an substantially pure pilus subunit protein derived from B. bronchiseptica and characterized by having a molecular weight of about 22,000 daltons as determined by sodium dodecyl sulfate-polyacrylamide gel electrophoresis and being cross-reactive with all purified pilus preparations from all B. bronchiseptica.

10. A composition of matter comprising an substantially pure pilus subunit protein derived from B. bronchiseptica and characterized by having a molecular weight of about 24,000 daltons as determined by sodium dodecyl sulfate-polyacrylamide gel electrophoresis and being cross-reactive with all purified pilus preparations from all B. bronchiseptica.

11. A composition of matter comprising substantially pure pilus subunit protein derived from B. bronchiseptica and characterized by having a molecular weight of about 21,000 and/or 22,000 and/or 24,000 daltons as determined by sodium dodecyl sulfate-polyacrylamide gel electrophoresis and being cross-reactive with all purified pilus preparations from all B. bronchiseptica.

12. A process for preparing the vaccine of any one of claims 1 to 7, which comprises mixing the protein with one or more physiologically acceptable excipients.

5488K                                                24170-FF

13.    A process for preparing the compositions of matter of Claim 8, 9, 10 or 11, which process comprises growing a strain of B. bronchiseptica in a suitable medium, separating the pili from the medium, and recovering the pilus subunit proteins.

24170-FF

-30-

CLAIMS:

1.    A process for preparing a vaccine comprising protein corresponding to one or more pilus subunit proteins derived from B. bronchiseptica, which process comprises mixing the protein with one or more physiologically acceptable excipients.

2.    A process of Claim 1, wherein there is produced a vaccine for immunization against B. bronchiseptica.

3.    A process according to Claim 1 or Claim 2 wherein said subunit protein is characterized by having a molecular weight of about 21,000 dalton and/or 22,000 dalton and/or 24,000 dalton as determined by sodium dodecyl sulfate-polyacrylamide gel electrophoresis (SDS-PAGE) and by being serologically cross-reactive with all purified pilus preparations from all B. bronchiseptica strains.

4.    The process of any one of claims 1,2 and 3 wherein said subunit protein is derived from the B. bronchiseptica strain NADL 2-9.

5.    A process of Claim 1 wherein there is produced a vaccine for immunization against B. pertussis induced whooping cough.

6.    The process of Claim 5 wherein the subunit protein has a molecular weight of about 22K as determined by sodium dodecyl sulfate-polyacrylamide gel electrophoresis (SDS-PAGE).

7.    A process of Claim 5 or 6 wherein said subunit protein is derived from the B. bronchiseptica strain 5389.

8. A process for preparing a composition of matter comprising pilus subunit protein derived from <u>B. bronchiseptica</u> and characterized by having a molecular weight of about 21,000 and/or 22,000 and/or 24,000 daltons as determined by sodium dodecyl sulfate-polyacrylamide gel electrophoresis and being cross-reactive with all purified pilus preparations from all <u>B. bronchiseptica</u>; which process comprises growing a strain of <u>B. bronchiseptica</u> in a suitable medium, separating the pili from the medium, and recovering the pilus subunit proteins.

9. A process according to Claim 8 wherein the composition of matter pilus subunit protein is characterized by having a molecular weight of about 21,000 daltons as determined by SDS-PAGE.

10. A process according to Claim 8 wherein the pilus subunit protein is characterized by having a molecular weight of about 22,000 daltons as determined by SDS-PAGE.

11. A process according to Claim 8 wherein the pilus subunit protein is characterized by having a molecular weight of about 24,000 daltons as determined by SDS-PAGE.

FIGURE I

WHITE BLOOD CELL COUNT

LEGEND

——— Tetanus toxoid

— — B. pertussis pilus

······· B. bronchiseptica pilus 5389

·— —· B. bronchiseptica pilus 2—9

········· Noninfected control

WHITE BLOOD CELL COUNT / MM$^3$ X 10$^3$

DAYS POSTCHALLENGE

1/2

0141671

FIGURE II

BODY WEIGHT GAIN

LEGEND

―――― Tetanus toxoid

― ― B. pertussis pilus

········· B. bronchiseptica pilus 5389

· ― · ― B. bronchiseptica pilus 2―9

·········· Noninfected control

% BODY WEIGHT GAIN

DAYS POSTCHALLENGE

2/2

0141671